# EUROPÄISCHE PATENTSCHRIFT

(11) **EP 0 379 223 B1**
(45) Veröffentlichungstag und Bekanntmachung des Hinweises auf die Patenterteilung: **04.08.1993**
(21) Anmeldenummer: 90101116.3
(22) Anmeldetag: 19.01.1990
(51) Int. Cl.: A61M 5/50

(54) **Einmalspritze**
Disposable syringe
Seringue à usage unique

(30) Priorität: 19.01.1989 DE 3901484
(43) Veröffentlichungstag der Anmeldung: 25.07.1990
(73) Patentinhaber: InterChem GmbH Gesellschaft für Biopolymere, W-8000 München 82 (DE)
(72) Erfinder: Kohler, Reinhard, D-8000 München 82 (DE)
(74) Vertreter: WILHELMS, KILIAN & PARTNER Patentanwälte

(56) Entgegenhaltungen:
- EP-A- 0 291 109
- EP-A- 0 345 159
- WO-A-90/03817
- FR-A- 2 606 643

## Beschreibung

Die Erfindung betrifft eine Einmalspritze mit einem Spritzenzylinder, an dem eine Kanüle vorgesehen ist und in dem ein relativ dazu dichtverschiebbarer Kolben angeordnet ist wie es im Oberbegriff des Anspruchs 1 angegeben ist.

Eine derartige Spritze ist aus des EP-A-0 291109 bekannt und wird zum Injizieren, beispielsweise von Medikamenten und anderen Stoffen, in den menschlichen Körper, beispielsweise in eine Körpervene, benutzt.

Aus hygienischen Gründen sowie aus Gründen der Vermeidung der Übertragung von Krankheiten ist es notwendig, daß eine Spritze nach ihrem Gebrauch sterilisiert wird, bevor sie erneut benutzt werden kann. Da die Möglichkeiten einer Sterilisation in dem dazu erforderlichen Grad Privatpersonen in der Regel nicht zur Verfügung stehen, sind diese auf sog. Einmalspritzen angewiesen, die dazu bestimmt sind, nur einmal benutzt und dann weggeworfen zu werden.

Es kommt jedoch immer wieder vor, daß auch derartige Einmalspritzen bewußt oder versehentlich nochmals benutzt werden, ohne daß die notwendige Sterilisation zwischen den beiden Benutzungen erfolgt ist.

Wenn zwei verschiedene Personen dieselbe Einmalspritze nacheinander benutzen, ohne sie dazwischen zu sterilisieren, besteht die Gefahr, daß Krankheitserreger zwischen den beiden Personen übertragen werden. Eine derartige Mehrfachbenutzung einer Einmalspritze sollte daher unter allen Umständen vermieden oder sogar ausgeschlossen werden.

Die der Erfindung zugrundeliegende Aufgabe besteht daher darin, die Einmalspritze nach dem Oberbegriff des Anspruchs 1 so auszubilden, daß keine Möglichkeit einer mehrfachen Benutzung gegeben ist.

Diese Aufgabe wird gemäß der Erfindung durch die Ausbildung gelöst, die im kennzeichnenden Teil des Anspruchs 1 angegeben ist.

Die erfindungsgemäße Einmalspritze ist somit so ausgebildet, daß sie sich bei ihrer Benutzung selbst zerstört, da nach dem erstmaligen Injizieren der zum Aufziehen der Spritze notwendige dichte Abschluß des Raumes zwischen dem Spritzenzylinder und dem Kolben zerstört wird, so daß die Spritze nicht nochmals aufgezogen und somit nur einmal benutzt werden kann.

Eine besonders bevorzugte Ausgestaltung und Weiterbildung der erfindungsgemäßen Einmalspritze ist Gegenstand des Anspruchs 2.

Im folgenden wird anhand der zugehörigen Zeichnung ein besonders bevorzugtes Ausführungsbeispiel der Erfindung näher beschrieben. Es zeigen
Fig. 1 eine Axialschnittansicht eines zylindrischen Hohlkörpers des Ausführungsbeispiels der erfindungsgemäßen Einmalspritze,
Fig. 2 eine teilweise geschnittene Seitenansicht des zugehörigen Stößels und
Fig. 3 eine Draufsicht auf die Stirnplatte des Stößels, gesehen von der dem Dorn abgewandten Seite.

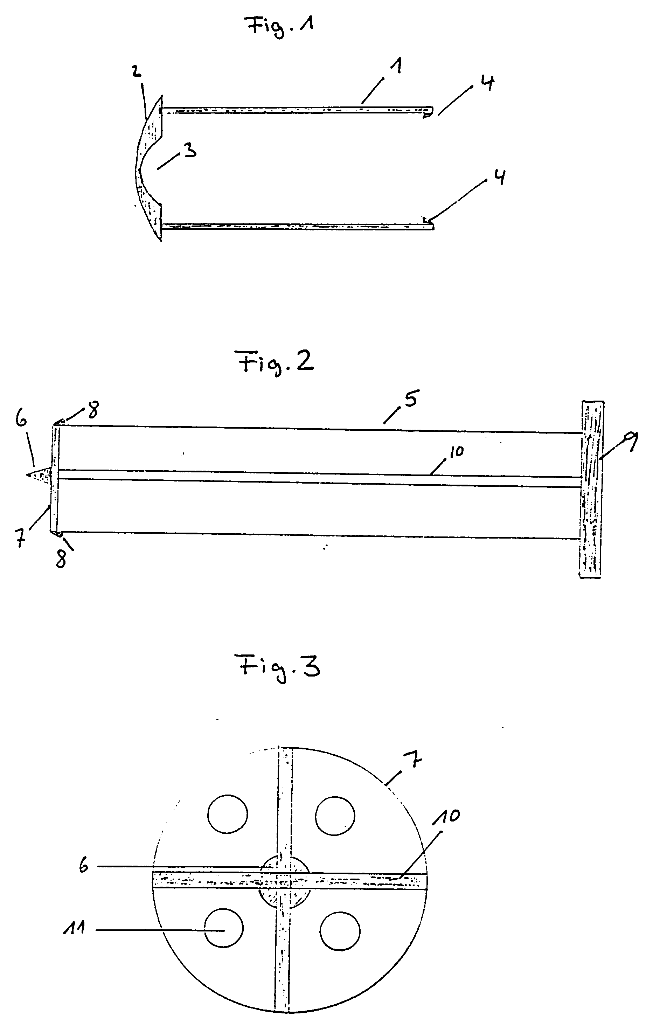

Der in der Zeichnung dargestellte Kolben des Ausführungsbeispiels der erfindungsgemäßen Einmalspritze, der in einem nicht dargestellten Spritzenzylinder relativ dazu dicht und axial verschiebbar angeordnet ist, besteht aus zwei Hauptteilen, nämlich einem zylindrischen Hohlkörper 1, der in Fig. 1 dargestellt ist und einem Stößel 5, der in den Fig. 2 und 3 dargestellt ist.

Der in Fig. 1 dargestellte zylindrischen Hohlkörper 1 ist an einer Stirnseite offen und an der anderen Stirnseite durch eine Stirnplatte 2 verschlossen, deren Außendurchmesser etwas größer als der Außendurchmesser des zylindrischen Hohlkörpers 1 ist. Mit den dadurch gebildeten Schultern liegt die Stirnwand 2 des zylindrischen Hohlkörpers 1 an der Innenwand des Spritzenzylinders dicht an, um für einen dichten Abschluß des Raumes zwischen dem Spritzenzylinder und dem Kolben, d.h. dem zylindrischen Hohlkörper 1 zu sorgen, der notwendig ist, um die Spritze aufziehen zu können.

Die Stirnwand 2 des zylindrischen Hohlkörpers 1 ist in ihrer Mitte schwächer als am Außenumfang ausgebildet, was beispielsweise dadurch erreicht werden kann, daß in der Mitte an der Innenseite der Stirnwand eine konkave Aussparung 3 vorgesehen ist. Vorzugsweise ist die Stirnwand 2 nach außen gewölbt ausgebildet und hat die konkave Aussparung 3 einen kleinen Krümmungsradius als diese Wölbung, so daß sich die gewünschte Verjüngung ergibt. Die Stirnwand 2 kann jedoch auch in anderer Weise in der Mitte schwächer ausgebildet sein. Beispiele dafür sind konvexe Aussparungen an der Außenseite, stufenförmige Ausnehmungen usw..

Wie es in Fig. 2 dargestellt ist, besteht der zugehörige Stößel 5, der im Innern des zylindrischen Hohlkörpers 1 angeordnet ist, aus einem für Einmalspritzen üblichen Bauteil in Form eines Kreuzstückes 10, das im Querschnitt kreuzförmig ausgebildet ist, und an seiner einen Stirnseite eine Stirnplatte 7 trägt, die so bemessen ist, daß sie die Querschnittsfläche des zylindrischen Hohlkörpers 1 im wesentlichen füllt. An der anderen Stirnseite des Kreuzstückes 10 ist eine Griffplatte 9 angebracht, deren Außendurchmesser vorzugsweise größer als der Außendurchmesser des Spritzenzylinders ist, so daß der Stößel 5 an der Griffplatte 9 bequem von Hand aus betätigt werden kann.

Die Stirnplatte 7 des Stößels 5 trägt an ihrer Außenseite in der Mitte einen Dorn 6 und ist in der in Fig. 3 dargestellten Weise mit Löchern 11 versehen, über die der Raum zwischen der Außenseite der Stirnplatte 7 und der Innenseite der Stirnwand 2 des zylindrischen Hohlkörpers 1 mit der Außenluft in Verbindung steht.

Arretiereinrichtungen 4, 8 sind in Form von Ansätzen, beispielsweise Ringansätzen, mit zueinander komplementärer Ausbildung an der Innenfläche des zylindrischen Hohlkörpers 1 an dessen offenen Ende sowie an der Außenkante der Stirnplatte 7 des Stößels 5 ausgebildet. Diese Arretiereinrichtungen 4 bilden gemeinsam einen Schnappverschluß.

Der zylindrische Hohlkörper 1 sowie der Stößel 5 können jeweils als einteilige Spritzgußteile aus dem gleichen Kunststoffmaterial, beispielsweise PVC, hergestellt sein. Der Dorn 6 kann dabei gleichfalls in einem Stück mit dem Stößel 5 geformt sein.

Im folgenden wird die Arbeitsweise des oben beschriebenen Ausführungsbeispiels der erfindungsgemäßen Einmalspritze beschrieben:

Im zusammengebauten Zustand ist der Stößel 5 im zylindrischen Hohlkörper 1 angeordnet, während dieser wiederum im Inneren des nicht dargestellten Spritzenzylinders sitzt. Die Anordnung des Stößels 5 im zylindrischen Hohlkörper 1 kann ohne Probleme aufgrund der diesen Bauteilen eigenen Elastizität erfolgen, da lediglich die Arretiereinrichtungen 4, 8 überwunden werden müssen, was durch eine entsprechende Schrägflächenausbildung der Ansätze 4 und 8 erleichtert sein kann.

Beim Aufziehen der Spritze wird der Stößel 5 zunächst mittels der Griffplatte 9 zurückgezogen, bis die an seiner Stirnplatte 7 vorgesehenen Arretiereinrichtungen 8 mit den Arretiereinrichtungen 4 im Inneren des zylindrischen Hohlkörpers 1 in Eingriff kommen. Ein weiteres Zurückziehen des Stößels 5 bewirkt, daß der zylindrischen Hohlkörper 1 mitgenommen wird und somit das zu injizierende Material in den Spritzenzylinder gesaugt wird. Dieser eigentliche Einsaugvorgang ist dann der gleiche wie beim Aufziehen einer herkömmlichen Einmalspritze.

Wenn die Spritze aufgezogen ist und die Injektion erfolgen soll, dann wird wie üblich zunächst das Restluftvolumen in der Kanüle dadurch beseitigt, daß eine geringe Menge des zu injizierenden Materials ausgespritzt wird. Dazu wird der Stößel 5 wiederum mittels des Handgriffes 9 in Richtung auf die Stirnwand 2 des zylindrischen Hohlkörpers 1 geschoben, wobei die Luft zwischen diesen Bauteilen durch die Löcher 11 in der Stirnplatte 7 des Stößels 5 entweicht. Am Ende dieser Vorschubbewegung erreicht der Dorn 6 die Innenfläche der Stirnwand 2 des zylindrischen Hohlkörpers 1. Ein weiteres Vorschieben des Stößels 5 bewirkt, daß der Dorn 6 in die Stirnwand 2 des zylindrischen Hohlkörpers 1 eindringt und diese durchstößt. Anschließend kommt die Stirnplatte 7 mit ihrer Außenfläche zur Anlage an der Innenfläche der Stirnwand 2. Die Ausbildung kann ohne Schwierigkeiten so gewählt werden, daß die Stirnwand 2 vom Dorn 6 dicht durchdrungen wird, so daß verhindert ist, daß das zu injizierende Material aus dem Innenraum des Spritzenzylinders entweicht.

Ein weiteres Vorschieben des Stößels 5 mittels der Griffplatte 9 führt dann dazu, daß auch der darauf sitzende Hohlkörper 1, dessen Stirnwand 2 durch den Dorn 6 durchdrungen ist, im Spritzenzylinder vorgeschoben wird und dadurch wie bei einer üblichen Spritze eine gewisse Menge des zu injizierenden Materials ausgestoßen wird. Anschließend kann die Kanüle eingestochen werden und kann durch ein weiteres Vorschieben des aus dem Hohlkörper 1 und dem Stößel 5 bestehenden Kolbens die Injektion durchgeführt werden.

Durch eine entsprechende Wahl des Materials und eine entsprechende Ausbildung des Dornes 6 des Stößels 5 einerseits sowie der Stirnwand 2 des zylindrischen Hohlkörpers 1 andererseits kann sichergestellt sein, daß der Durchdringungsdruck, d.h. der Druck, der notwendig ist, damit der Dorn 6 die Stirnwand 2 durchdringt, kleiner als der zum Injizieren notwendige Druck ist, so daß der Dorn 6 die Stirnwand 2 vor dem eigentlichen Injektionsvorgang durchstößt.

Da vor oder spätestens während des Injektionsvorganges somit die Stirnwand 2 des zylindrischen Hohlkörpers 1 vom Dorn 6 durchstoßen und damit zerstört wird, kann die Spritze nach einer einmaligen Injektion nicht nochmals benutzt werden, da bei dem Versuch, die bereits benutzte Spritze aufzuziehen, der Raum im Spritzenzylinder durch die vom Dorn 6 in der Stirnwand 2 des zylindrischen Hohlkörpers 1 gebildete Öffnung Luft ziehen würde. Somit ist zumindest eine unbeabsichtigte erneute Benutzung der Einmalspritze ausgeschlossen.

## Patentansprüche

1. Einmalspritze mit einem Spritzenzylinder, an dem eine Kanüle vorgesehen ist und in dem ein relativ dazu dicht verschiebbarer Kolben angeordnet ist, wobei der Kolben aus einem zylindrischen Hohlkörper (1), der an einer Stirnseite offen und an der anderen Stirnseite durch eine Stirnwand (2) geschlossen ist, die in der Mitte (3) schwächer als am Umfangsbereich ausgebildet ist, und einem Stößel (5) besteht, der im zylindrischen Hohlkörper (1) relativ dazu verschiebbar angeordnet ist und an seiner der Stirnwand (2) des zylindrischen Hohlkörpers (1) zugewandten Stirnseite einen Dorn (6) trägt, eine Arretiereinrichtung (4, 8) zum Arretieren des Stößels (5) am zylindrischen Hohlkörper (1) in einem Abstand von der Stirnwand (2) des zylindrischen Hohlkörpers (1) am zylindrischen Hohlkörper (1) und/oder am Stößel (5) vorgesehen ist und der Raum zwischen der Stirnwand (2) des zylindrischen Hohlkörpers (1) und der Stirnseite des Stößels (5) mit der Außenluft in Verbindung steht, dadurch gekennzeichnet, daß an der Stirnseite des Stößels (5) eine Stirnplatte (7) vorgesehen ist, die die Querschnittsfläche des Innenraums des zylindrischen Hohlkörpers (1) im wesentlichen einnimmt und in der wenigstens ein Loch (11) vorgesehen ist, über das der Raum zwischen der Stirnwand (2) des zylindrischen Hohlkörpers ,(1) der Stirnplatte (7) mit der Außenluft verbunden ist.

2. Einmalspritze nach Anspruch 1, dadurch gekennzeichnet, daß die Arretiereinrichtung (4, 7) aus zwei zueinander komplementär ausgebildeten Ansätzen besteht, die an der Innenfläche des zylindrischen Hohlkörpers (1) und an der Außenkante der Stirnplatte (7) des Stößels (5) jeweils vorgesehen sind.

## Claims

1. Single use syringe with a syringe cylinder on which a hollow needle is provided, and in which a piston is arranged which is sealingly movable relative thereto, the piston comprising a cylindrical hollow body (1) open on one end and closed on the other end by a front wall (2) formed weaker in the mid-section (3) than in the peripheral area, and a plunger (5) arranged in the cylindrical hollow body (1) relatively moveable relative thereto and carrying in its front side facing the front wall (2) of the cylindrical hollow body a punch (6), an arresting device (4, 8) for arresting the plunger (5) at the cylindrical hollow body (1) at a distance from the front wall (2) of the cylindrical hollow body (1) being provided on the cylindrical hollow body (1) and/or on the plunger (5), and the space between the front wall (2) of the cylindrical hollow body (1) and the front side of the plunger (5) being connected to the outside air, characterized in that at the front side of the plunger (5) a front plate (7) is provided, which essentially occupies the cross-sectional area of the interior of the cylindrical hollow body (1) and in which there is provided at least one hole (11), via which the space between the front wall (2) of the cylindrical hollow body (1) of the front plate (7) is connected to the outside air.

2. Single use syringe according to claim 1, characterized in that the arresting device (4, 7) consists of two complementary shoulders respectively provided on the inside surface of the cylindrical hollow body (1) and on the outside edge of the front plate (7) of the plunger (5).

## Revendications

1. Seringue à usage unique avec un corps de pompe auquel est fixé une canule et dans lequel est placé un piston qui peut s'y déplacer de manière étanche et dont le piston est constitué d'un cylindre (1) creux avec un bout ouvert et avec un autre bout fermé par un paroi (2) dont le centre (3) est plus faible que la périphérie et d'un coulisseau (5) qui est monté mobile dans ce cylindre creux (1) et qui porte un stylet (6) à son bout correspondant à la paroi (2) du cylindre creux (1) et où ce cylindre creux (1) et/ou coulisseau (5) est (sont) pourvu(s) d'un dispositif d'immobilisation (4, 8) pour retenir le coulisseau (5) dans le cylindre creux (1) à distance de la paroi (2) de ce dernier (1), l'espace entre la paroi (2) du cylindre creux (1) et le bout du coulisseau (5) étant en communication avec l'atmosphère, seringue caractérisée en ce que ce bout du coulisseau (5) est muni d'un plateau (7) qui correspond pratiquement à l'aire de l'intérieur du cylindre creux (1) et qui est pourvu d'au moins un trou (11) par l'entremise duquel l'espace entre la paroi (2) du cylindre creux (1) et le plateau (7) est mis en communication avec l'atmosphère.

2. Seringue selon la revendication 1, caractérisée en ce que le dispositif d'immobilisation (4, 8) est constitué de deux appendices complémentaires l'un de l'autre qui sont disposés l'un sur la surface intérieure du corps creux (1) et l'autre au bord extérieur du plateau (7) du coulisseau (5).
